# EUROPEAN PATENT APPLICATION

(11) **EP 2 979 620 A1**
(43) Date of publication of application: **03.02.2016**
(21) Application number: 13880686.4
(22) Date of filing: 27.03.2013
(51) Int. Cl.: A61B 3/10

(54) **OPHTHALMIC MOISTURE MEASUREMENT INSTRUMENT AND OPHTHALMIC MOISTURE MEASUREMENT METHOD**

(71) Applicant: Kabushikikaisha Raifu, Koshigaya-shi, Saitama 343-0846 (JP); Taisei Co. Ltd., Chichibu-shi, Saitama 369-1593 (JP); Tsubota Laboratory Incorporated, Tokyo (JP)
(72) Inventor: FURUKAWA, Makoto, Koshigaya-shi Saitama 343-0846 (JP); SATO, Ryuki, Kitakatsushika-gun Saitama 343-0111 (JP); NITTA, Katsumi, Chichibu-shi Saitama 369-1593 (JP); KOSUGE, Haruo, Chichibu-shi Saitama 369-1593 (JP); MIYAHARA, Takayuki, Chichibu-shi Saitama 369-1593 (JP); KAWASHIMA, Motoko, Tokyo 123-0851 (JP)
(74) Representative: TBK
(86) International application number: PCT/JP2013/059071
(87) International publication number: WO 2014/155577

(57) **Abstract**

An apparatus for measuring eye's moisture 1 includes: a detection surface 30 placed on a position in contact with a conjunctiva, a sclera, or a cornea or a position opposed to a conjunctiva, a sclera, or a cornea; and a capacitance sensor 40 provided on the detection surface 30. As a result, an apparatus and a method for measuring eye's moisture that are capable of simply and objectively measuring the state of tear fluid are provided.

## Description

### Field

The present invention relates to an apparatus and a method for measuring the state of eye's moisture, that is, the state of tear fluid on and around the surface of an eyeball.

### Background

For diagnosis of dry eye or the like, a Schirmer's test, a phenol red thread test, a tear film breakup time (BUT) test, and the like are used as a method of measuring the state of tear fluid on and around the surface of an eyeball.

In the Schirmer's test, a long narrow strip of filter paper is placed between the eyelids for several minutes and the length of the wet part that has absorbed tear fluid in the filter paper is measured. In the phenol red thread test, a cotton thread is used instead of the filter paper (for example, see Patent Document 1).

In the tear film breakup time test, a time until the tear film is broken up to cause a dry spot is measured during no blink condition. The breakup of the tear film is visually distinguished by previously staining tear fluid with a fluorescein solution.

### Citation List

### Patent Literature

Patent document 1: JP, 06-154165, A

### Summary

### Technical Problem

The Schirmer's test and the tear film breakup time test, however, have no established consistent approach for measurement as evident from the fact that there are variations on a facility basis, for example, and thus have a problem of difficulty to objectively take measurements. Specifically, the Schirmer's test has no well-defined procedures, including how a piece of filter paper should be held and whether or not tear fluid should be wiped off before the measurement, and depending on how the filter paper is held, the secretion of tear fluid may be promoted as stimulated by the filter paper, rendering it difficult to objectively determine the state of tear fluid.

In the tear film breakup time test, the definition is unclear on when and in what state the tear film is determined to be broken, and the determination of the breakup of the tear film may vary depending on the amount and the concentration of the fluorescein solution used. Therefore, the tear film breakup time test also has difficulty to objectively take measurements.

In view of these factual problems, the present invention is to provide apparatus and a method for measuring eye's moisture that are capable of easily and objectively measuring the state of tear fluid.

### Solution to Problem

(1) The present invention is an apparatus for measuring eye's moisture, including: a detection surface placed on a position in contact with a conjunctiva, a sclera, or a cornea or a position opposed to a conjunctiva, a sclera, or a cornea; and a capacitance sensor provided on the detection surface.
(2) In the apparatus for measuring eye's moisture of the above-described means, the present invention is also characterized in that further including a gripper having an approximate-rod shape, the gripper being connected with the detection surface, and an angle between the longitudinal direction of the gripper and the detection surface is from 0 to 45°.
(3) In the apparatus for measuring eye's moisture of the above-described means, the present invention is also characterized in that further including a detachable part detachably connected with one end in the longitudinal direction of the gripper, and the detection surface is provided on the detachable part.
(4) In the apparatus for measuring eye's moisture of the above-described means, the present invention is also characterized in that the detachable part has a curved shape which gradually decreases its cross-sectional area from the gripper to the detection surface.
(5) In the apparatus for measuring eye's moisture of the above-described means, the present invention is also characterized in that the detection surface is disposed at an outer peripheral side of the outer peripheral surface of the gripper.
(6) In the apparatus for measuring eye's moisture of the above-described means, the present invention is also characterized in that the detection surface has a circular or an elliptical shape.
(7) In the apparatus for measuring eye's moisture of the above-described means, the present invention is also characterized in that further including: a controller controlling the sensor; a display displaying a measurement result from the sensor; and a power supply supplying electric power to the controller and the display, and the controller, the display, and the power supply are provided in the gripper.
(8) In the apparatus for measuring eye's moisture of the above-described means, the present invention is also characterized in that further including: a cover having an approximate-cup shape, the cover being disposed so as to bring the opening edge of the cover into contact with a subject's face, and the detection surface is provided inside the cover.
(9) In the apparatus for measuring eye's moisture of the above-described means, the present invention is also characterized in that the detection surface is provided at the bottom of the cover.
(10) In the apparatus for measuring eye's moisture of the above-described means, the present invention is also characterized in that the cover is provided with a sensor attaching member to which the sensor is attached; and a contact member to be brought into contact with a subject's face, and the sensor attaching member and the contact member are detachably connected with each other.
(11) In the apparatus for measuring eye's moisture of the above-described means, the present invention is also characterized in that the sensor is provided with a substrate disposed along the detection surface; and a first electrode and a second electrode, both of which are formed on the same face of the substrate, and at least one linear gap is provided between the first electrode and the second electrode.
(12) In the apparatus for measuring eye's moisture of the above-described means, the present invention is also characterized in that the first electrode and the second electrode are each provided with an adjoining part, the adjoining parts adjoining across the linear gap, and the adjoining part is formed in a shape having a width in a distance direction of the linear gap that is greater than the distance of the linear gap.
(13) The present invention is also a method for measuring eye's moisture, characterized in that a detection surface on which a capacitance sensor is provided is located at a position in contact with a conjunctiva, a sclera, or a cornea or a position opposed to a conjunctiva, a sclera, or a cornea.

### Advantageous Effects of Invention

The apparatus and the method for measuring eye's moisture according to the present invention have a beneficial effect of easily and objectively measuring the state of tear fluid.

### Brief Description of Drawings

Fig. 1 is a perspective view showing the appearance of an apparatus for measuring eye's moisture according to a first embodiment of the present invention.
Fig. 2(a) is a plan view of the apparatus for measuring eye's moisture.
Fig. 2(b) is a front view of the apparatus for measuring eye's moisture.
Fig. 2 (c) is a left side view of the apparatus for measuring eye's moisture.
Fig. 3(a) is a front view of the detachable part.
Fig. 3(b) is a bottom view of the detachable part.
Fig. 4(a) is a schematic diagram showing a structure of the sensor.
Fig. 4 (b) is a schematic diagram showing one example of another structure of the sensor.
Figs. 5 (a) to 5(c) are schematic diagrams showing an arrangement example of the sensor.
Fig. 6 is a diagram showing one example of a state during the use of the apparatus for measuring eye's moisture.
Fig. 7 is a perspective view showing the appearance of the apparatus for measuring eye's moisture according to a second embodiment of the present invention.
Fig. 8(a) is a front view of the cover.
Fig. 8(b) is a cross-sectional view taken along a line A-A in Fig. 8(a).
Fig. 9 is a diagram showing one example of a state during the use of the apparatus for measuring eye's moisture.

### Description of Embodiments

Embodiments of the present invention will be explained below with reference to the accompanying drawings.

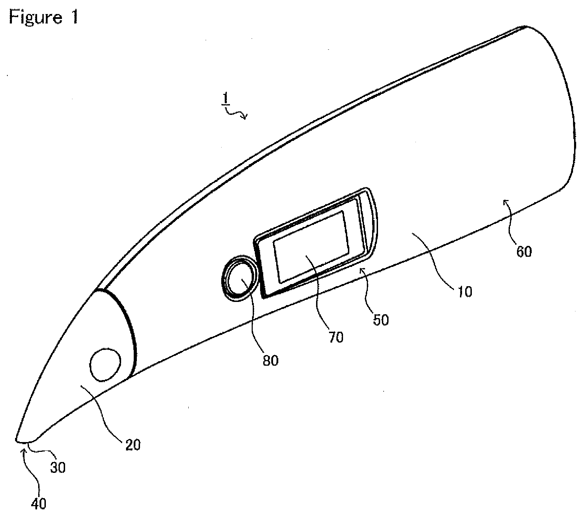

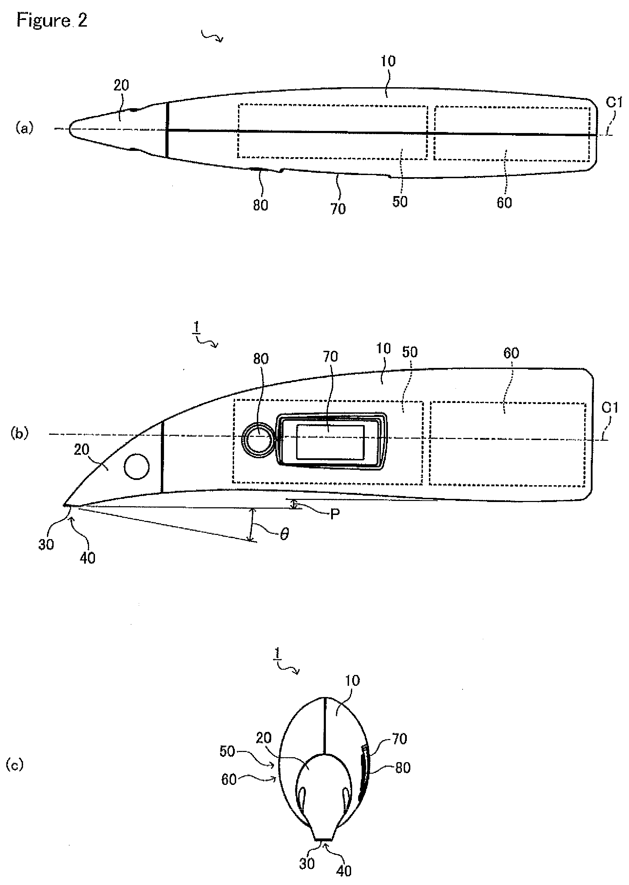

First, an apparatus for measuring eye's moisture 1 according to a first embodiment of the present invention will be explained below. Fig. 1 is a perspective view showing the appearance of an apparatus for measuring eye's moisture 1 according to this embodiment. Fig. 2(a) is a plan view of the apparatus for measuring eye's moisture 1. Fig. 2(b) is a front view of the apparatus for measuring eye's moisture 1. Fig. 2 (c) is a left side view of the apparatus for measuring eye's moisture 1.

As these figures shows, the apparatus for measuring eye's moisture 1 is provided with a gripper 10 having an approximate-rod shape; a detachable part 20 attached to one end of the gripper 10; a detection surface 30 provided in the detachable part 20; a sensor 40 disposed on the detection surface 30; a controller 50 and a power supply 60, both of which are disposed in the gripper 10; and a display 70 and an operation button 80, both of which are disposed on the front side of the gripper 10.

The gripper 10 is gripped by a measurer at the time of measurement. As Fig. 2 (a) shows, the gripper 10 has an approximate-spindle shape from the planar view, and curves slightly and is gradually reduced in height to the detachable part 20. The apparatus for measuring eye's moisture 1 of this embodiment is used with the detection surface 30 being closer to or in contact with to an eyeball at the time of measurement. Thus, the gripper 10 and the detachable part 20 have a shape with a soft curved line and a soft curved surface so as not to provide a subject with a sense of fear. In this embodiment, since the controller 50, the power supply 60, the display 70, and the operation button 80 are provided in the gripper 10, the entire device is kept compact and measurements can be taken simply and easily without being bothered with cables or the like.

The detachable part 20 has a detection surface 30 in contact with a subject. The detachable part 20 is detachably attached to the gripper 10 and thus can be thrown away as a so-called disposable. The detachable part 20 has a curved shape which gradually decreases its cross-sectional area from the gripper 10 to the detection surface 30 and has an outer peripheral surface forming a curved surface continued from the gripper 10. This allows to alleviate the subject's sense of fear while downsizing the detection surface 30 to be easily brought into contact with a narrow area in a subject's eye.

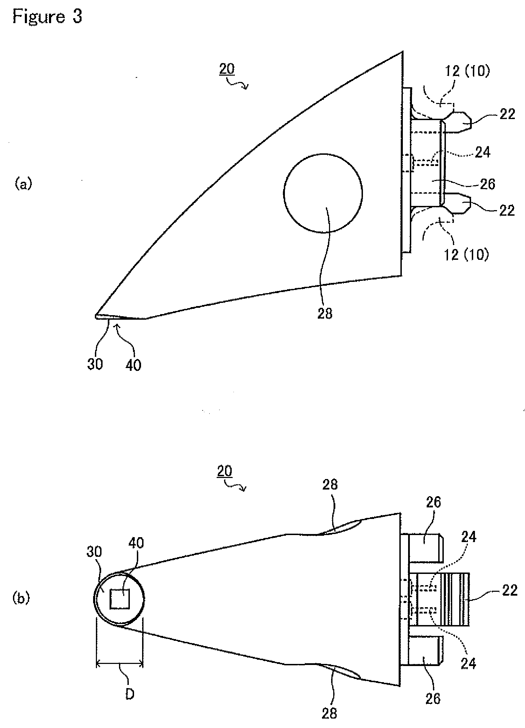

Fig. 3 (a) is a front view of the detachable part 20. Fig. 3 (b) is a bottom view of the detachable part 20. As these figures show, the detachable part 20 is provided with two locking pieces 22 on the end of the gripper 10 side. These locking pieces 22 are locked to the locking parts 12 provided on the gripper 10, so as to attach the detachable part 20 to the gripper 10. Between the two locking pieces 22, two connector terminals 24 with a rod shape, both of which are electrically connected with the sensor 40, are provided. Specifically, these two connector terminals 24 are connected with the corresponding terminals on the gripper 10 by attaching the detachable part 20 to the gripper 10. As a result, the sensor 40 and the controller 50 are electrically connected with each other.

On the end of the gripper 10 side of the detachable part 20, two projections 26 are also provided for positioning the detachable part 20 in relation to the gripper 10 and protecting the connector terminals 24. The two circular concaves 28 provided on the outer peripheral surface of the detachable part 20 are to prevent a slip when the detachable part 20 is attached and detached by placing fingers on.

The detection surface 30 is to be brought into contact with the conjunctiva, the sclera, or the cornea of a subject's eye. As Fig. 2(b) shows, the detection surface 30 is formed in approximately parallel with a longitudinal axial center C1 of the gripper 10 and also disposed at an outer peripheral side of the outer peripheral surface of the gripper 10. In this embodiment, such a structure of the detection surface 30 allows the apparatus for measuring eye's moisture 1 to be moved in a direction orthogonal to the axial center C1 of the gripper 10 so as to bring the detection surface 30 into contact with a conjunctiva or the like. This allows to erase the image of a subject's eye to be stabbed with a rod and to alleviate the subject's sense of fear. As a result, the measurement is carried out smoothly.

The projection length P from the outer peripheral surface of the gripper 10 at the detection surface 30 is not limited in particular. However, the projection length P is preferably within a range from 0.5 to 10 mm, more preferably within a range from 2 to 8 mm, most preferably within a range from 3 to 4 mm, in order to bring the detection surface 30 into contact with a subject's conjunctiva or the like smoothly without providing a subject with a sense of fear. The detection surface 30 is not limited be in parallel with the axial center C1 of the gripper 10 but may have an angle to the axial center C1. However, the angle θ between the detection surface 30 and the axial center C1 of the gripper 10 is preferably within a range from 0 to 45 ° , more preferably within a range from 0 to 30°, most preferably within a range from 0 to 10°, in view of the subject's sense of fear.

The detection surface 30 may have any shapes with no particular limitation but preferably a non-angular shape. In this embodiment, the detection surface 30 has an approximate-circle shape as Fig. 3(b) shows. The detection surface 30 with a circular or an elliptical shape decreases the possibility of damaging a conjunctiva or the like to enhance the safety of the measurement. The outer periphery of the detection surface 30 is made rounded appropriately.

The size of the detection surface 30 is not limited in particular but preferably as small as possible to expand the accessible range to carry out a more accurate and more objective measurement. Specifically, the diameter (or length of the major axis of the ellipse) D of the detection surface 30 is preferably 6 mm or less, more preferably 5 mm or less, most preferably 4 mm or less.

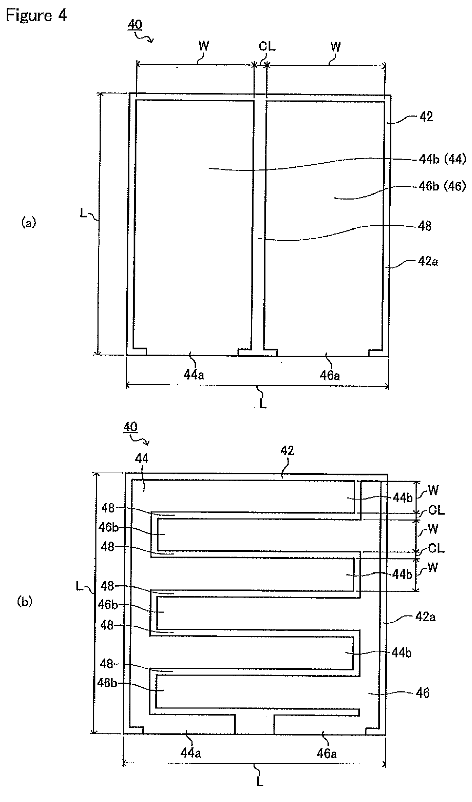

The sensor 40 is a capacitance sensor, which is disposed on the detection surface 30 as Fig. 3(b) shows. Fig. 4(a) is a schematic diagram showing a structure of the sensor 40. As this figure shows, the sensor 40 is provided with a substrate 42 which is an insulator with an approximate-square plate shape, and a first electrode 44 and a second electrode 46, both of which are conductive thin films formed on the surface 42a of the substrate 42. Accordingly, the sensor 40 is to measure an amount of moisture as a conductor existing around the sensor 40 based on the variation of the electrostatic capacity between the first electrode 44 and the second electrode 46.

In this embodiment, the first electrode 44 and the second electrode 46 are respectively provided with terminals 44a and 46a, both of which are electrically connected with the corresponding connector terminals 24. Furthermore, the first electrode 44 and the second electrode 46 are respectively provided with adjoining parts 44b, 46b, both of which adjoin each other holding a linear gap 48 therebetween. In the first electrode 44 and the second electrode 46, an electrode width W at the adjoining part 44b and an electrode width W at the adjoining part 46b are greater than an interval CL between the adjoining parts 44b and 46. More specifically, the electrode width W which is a dimension in a direction of a distance CL of the linear gap 48 at the adjoining parts 44b and 46b is greater than the distance CL of the linear gap 48.

In this embodiment, such structure of the first electrode 44 and the second electrode 46 allows to downsize the sensor 40 without lowering the detection sensitivity of moisture. As a result, the length L of one side of the substrate 42 can be reduced to 2 mm or less. This achieves the detection surface 30 downsized to the extent that the detection surface 30 can be easily brought into contact with a conjunctiva or the like in an eye. Specifically, in the sensor 40 in this embodiment, the length L of one side of the substrate 42 is 2 mm, the electrode width W at the adjoining parts 44b of the first electrode 44 and the electrode width W at the adjoining parts46b of the second electrode 46 are 0.9 mm, the interval CL between the adjoining parts 44b and 46b is 0.1 mm. This ensures a sufficient detection sensitivity with the detection surface 30 having a diameter of 4 mm or less.

Fig. 4(b) is a schematic diagram showing one example of another structure of the sensor 40. As this figure shows, the first electrode 44 and the second electrode 46 may have strip-shaped adjoining parts 44b and 46b arranged like comb teeth. Even in this case, by setting the electrode width W of the adjacent part 44b and the electrode width W of the adjacent part 46b to be greater than the interval CL between the adjoining parts 44b and 46b, a sufficient detection sensitivity is ensured with decreasing the length L of the one side of the substrate 42 to 2 mm or less.

The inventors of the present application measured an electrostatic capacity C_{D} in the dry state and an electrostatic capacity C_{W} in the wet states for the sensor 40 in this embodiment shown in Fig. 4(a) and determined the ratio C_{D}/C_{W} in accordance with JIS C5101-8. The inventors thus obtained C_{D}=0.29(pF) and C_{W}=2.3(pF) resulting in C_{D}/C_{W}=0.13. This result shows the extremely excellent sensitivity of the detection. Here, the electrostatic capacity C_{D} in the dry state is measured at normal temperature and humidity (temperature: 25°C, humidity: 65%RH). The electrostatic capacity C_{W} in the wet state is measured in a state in which the sensor face is in contact with a dust-free paper towel containing a 1.5% salt solution. The size of the sensor 40 measured is same as described above. The frequency of the input voltage for the capacitance measurement is 100 kHz.

The inventors of the present application also carried out the measurement for the sensor 40 having the structure shown in Fig. 4 (b) in the same way and thus obtained C_{D}=0. 8 (pF) and C_{W}=1.9 (pF) resulting in C_{D}/C_{W}=0.42. This result shows the extremely excellent sensitivity of the detection for the sensor 40 having this structure. In the sensor 40 having the structure shown in Fig. 4(b) in this measurement, the length L of one side of the substrate 42 is 2 mm, the electrode width W at the adjoining part 44b of the first electrode 44 and the electrode width W at the adjoining part 46b of the second electrode 46 are 0.2 mm, the interval CL between the adjoining parts 44b and 46b is 0.05 mm.

According to the findings from the study of the inventors of the present application, the ratio W/CL of the electrode width W to the interval CL is preferably greater than 1 and is equal to or smaller than 10. The interval CL is preferably within a within a range from 0.05 to 0.2, and the electrode width W is preferably within a range 0.1 to 2 mm.

As the material of the substrate 42 of the sensor 40, appropriate materials such as resin and ceramics can be used. As the material of the first electrode 44 and the second electrode 46, appropriate materials such as copper and aluminum can be used. To improve the corrosion resistance of the sensor 40, the surface of the first electrode 44 and the second electrode 46 may be plated with gold or the like. Furthermore, the first electrode 44, the second electrode 46, and the surface 42a of substrate 42 may be coated with an insulating resin, etc. In other words, this coating prevents the first electrode 44 and the second electrode 46 from short-circuiting so that the sensor 40 may be brought into direct contact with a conjunctiva or the like.

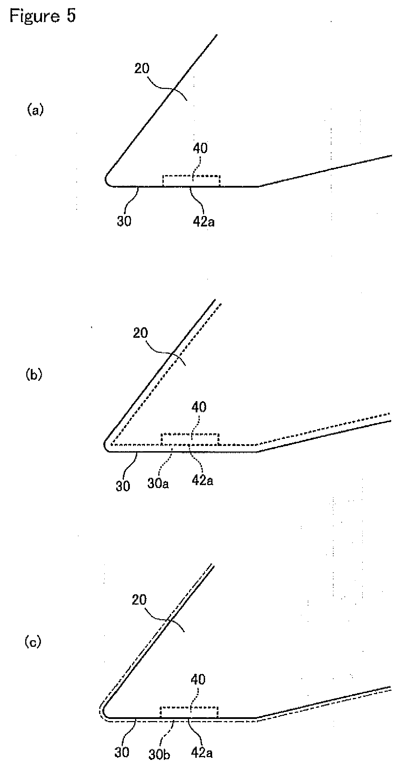

Figs. 5 (a) to 5(c) are schematic diagrams showing an arrangement example of the sensor 40. For example, the sensor 40 may be disposed in a state in which the surface 42a having the first electrode 44 and the second electrode 46 formed thereon is exposed and the surface 42a is flushed with the detection surface 30 as Fig. 5(a) shows. Alternatively, the sensor 40 may be disposed inside the outer wall 30a which is made of a resin or the like and forms the detection surface 30 as Fig. 5(b) shows. Furthermore, the exposed surface 42a may be covered with a resin film 30b or the like from the outside as Fig. 5(c) shows.

This means that the sensor 40 may be or may not be brought into direct contact with a conjunctiva or the like as long as the first electrode 44 and the second electrode 46 are prevented from short-circuiting. In other words, an appropriate insulator (dielectric substance) including the above-mentioned coating only has to be disposed between the sensor 40 and a conjunctiva or the like.

In this embodiment, the moisture can be measured by using a capacitance sensor 40 without being direct contact with a conjunctiva or the like. Thus, the arrangement, the covering state or the like of the sensor 40 are appropriately set so that the measurement is carried out under proper hygienic condition according to the purpose pf use, the use environment or the like. Moreover, in this embodiment, by using the sensor 40 as a disposable with the detachable part 20, the wash, the sterilization or the like can be omitted as much as possible so as to easily obtain proper hygienic condition. Needless to say, the detachable part 20 may be washed or sterilized for reuse after the measurement. In this case, this embodiment allows the detachable part 20 to be easily washed and sterilized after detached from the gripper 10.

Returning to Figs. 1 and 2(a) to 2(c), the controller 50 has a known configuration provided with an appropriate microcomputer chip and the like to control the sensor 40 and the display 70. The controller 50 also calculates the amount of moisture as a measurement result based on a signal from the sensor 40 and various indices or the like based on the amount of moisture and displays these calculation results on the display 70. The power supply 60 can accommodate a dry cell battery or a rechargeable battery to supply electric power to the controller 50, the display 70, and the like. The display 70 is composed of a liquid crystal panel or the like to display various kinds of information such as the measurement result or the like. The operation button 80 is to carry out various kinds of operation such as turning ON/OFF the power supply, switching the modes or the like.

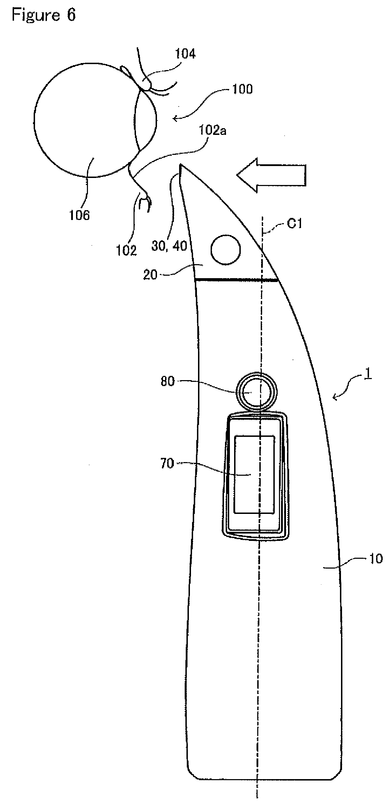

Next, the use of the apparatus for measuring eye's moisture 1 will be explained below. Fig. 6 is a diagram showing one example of a state during the use of the apparatus for measuring eye's moisture 1. Before the measurement of eye's moisture with the apparatus for measuring eye's moisture 1, a new detachable part 20 (or a washed and sterilized detachable part 20) is attached to the gripper 10. Subsequently, the operation button 80 is operated to turn on the power supply of the apparatus for measuring eye's moisture 1 and switch to the measurement mode.

After the apparatus for measuring eye's moisture 1 is switched to the measurement mode, the gripper 10 is gripped to move the apparatus for measuring eye's moisture 1 in a direction approximately orthogonal to the axial center C1 of the gripper 10, and then the detection surface 30 is brought into closer to an subject's eye 100 to bring the detection surface 30 into contact with any site of the eye 100, as Fig 6 shows. At this time, the gripper 10 and a measurer's hand holding the gripper 10 are on the edge of or off the subject's sight so that the subject can allow the detection surface 30 to be put into the subject's eye without any resistance.

The site with which the detection surface 30 is to be brought into contact is suitably a conjunctiva 102a (including tarsal conjunctiva and fornix conjunctiva) in a lower eyelid 102 as Fig. 6 shows. Specifically, the conjunctiva 102a inside a lower eyelid 102 can be relatively easily exposed by turning over the lower eyelid 102 with finger or the like. Furthermore, the conjunctiva 102a is less sensitive to pain when in contact with the detection surface 30. Therefore, the detection surface 30 can be smoothly brought into contact with the conjunctiva 102a.

Once in contact with a conjunctiva 102a, the detection surface 30 is maintained in contact with the conjunctiva 102a for only a predetermined time (for example, from 2 to 3 seconds) and then removed from the conjunctiva 102a. The measurement with the sensor 40 is now completed, and the amount of moisture as a measurement result and various indices or the like are displayed on the display 70. As described above, the apparatus for measuring eye's moisture 1 according to this embodiment can rapidly and easily carry out the measurement compared with the conventional Schirmer's test or the like. Moreover, since the amount of moisture as a measurement result and various indices or the like based on the amount of moisture can be displayed in numerical values, the objective measurement can be made.

The site with which the detection surface 30 is to be brought into contact is suitably a conjunctiva 102a inside a lower eyelid 102 as described above but may be another site. In other words, the site with which the detection surface 30 is to be brought into contact may be a conjunctiva (including tarsal conjunctiva and fornix conjunctiva) inside an upper eyelid 104 or may be a bulbar conjunctiva, a sclera, or a cornea on the surface of an eyeball 106. Moreover, the detection surface 30 may be brought into contact with an excretory duct connected with a lacrimal gland, an upper and a lower lacrimal punctum, a lacrimal caruncle or the like. The measurement of the amount of moisture at various sites as described above may lead to more versatile evaluation and diagnosis.

When the detection surface 30 is brought into contact with a site, the posture of the apparatus for measuring eye's moisture 1 is not limited to the posture with the axial center C1 of the gripper 10 being substantially vertical, and may be any posture with the axial center C1 being horizontal or angled.

Next, the apparatus for measuring eye's moisture 2 according to a second embodiment of the present invention will be explained below. The apparatus for measuring eye's moisture 2 according to this embodiment is to measure the evaporation amount of moisture, that is, the evaporation amount of tear fluid. The basic structure is similar to that of the apparatus for measuring eye's moisture 1 according to the first embodiment. Therefore, in the second embodiment, the like reference signs are assigned to the like parts as those in the first embodiment. The description of like parts will be omitted, and only the different parts will be explained below.

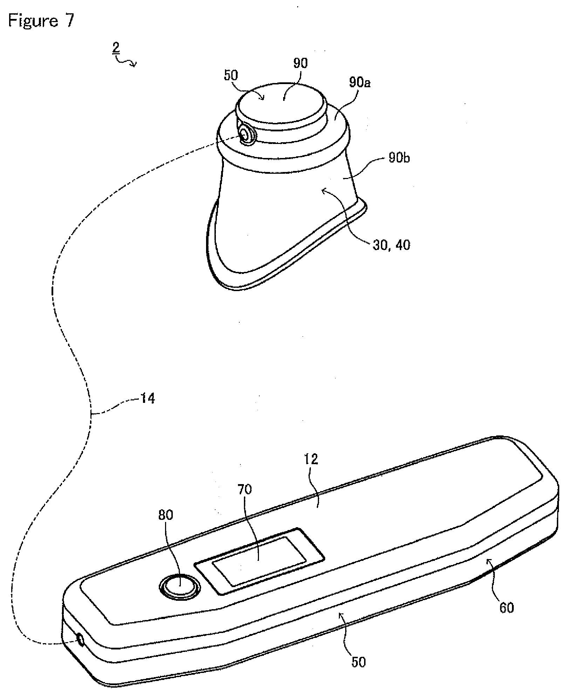

Fig. 7 is a perspective view showing the appearance of the apparatus for measuring eye's moisture 2 according to this embodiment. As this figure shows, the apparatus for measuring eye's moisture 2 is provided with a main body 12 and a cover 90 connected with the main body 12 through a cable 14 that is simply illustrated. The main body 12 accommodates a part of the controller 50 and a power supply 60 inside and is provided with a display 70 and an operation button 80.

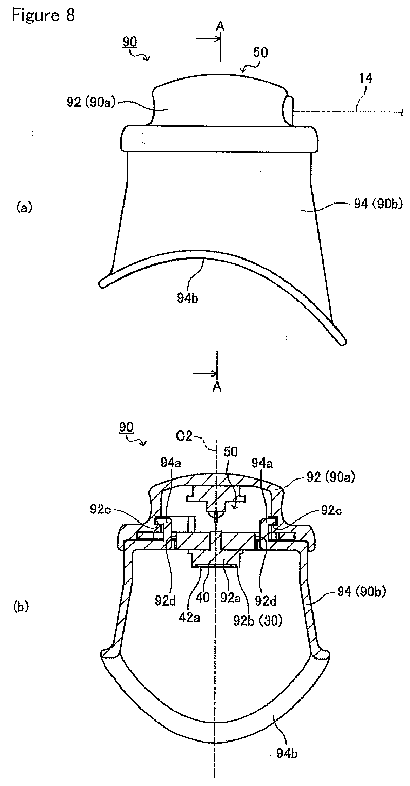

The cover 90 consists of a bottom 90a and a skirt 90b and has an approximate-cup shape like a swimming goggle. The detection surface 30 is provided inside the bottom 90a. Fig. 8(a) is a front view of the cover 30. Fig. 8(b) is a cross-sectional view taken along a line A-A in Fig. 8 (a). As these figures show, the cover 90 is provided with an approximately-discoid sensor attaching member 92 having a flange, the sensor attaching member 92 forming the bottom 90a; and an approximately-cylindrical contact member 94 connected with the sensor attaching member 92, the contact member 94 forming the skirt 90b.

The sensor attaching member 92 is provided with an approximately-rectangular projecting part 92a in the center, in which the projecting part projects toward the inside of the contact member 94. The sensor 40 is provided on the apical surface 92b of the projecting part 92a. Therefore, the apical surface 92b of the projecting part 92a forms the detection surface 30. Although not shown, a part of the circuit forming the controller 50, such as an oscillation circuit, an oscillation detection circuit and the like to detect the variation of the electrostatic capacity in the sensor 40 are arranged in the sensor attaching member 92. These circuits and the sensor 40 are electrically connected with the controller 50 in the main body 12 through the cable 14 connected with the main body 12.

The contact member 94 is detachably connected with the sensor attaching member 92 by locking the locking piece 94a provided at the one end of the contact member 94 to a locking part 92c of the sensor attaching member 92. Specifically, the locking piece 94a is inserted in the insertion hole 92d of the sensor attaching member 92, and then the contact member 94 is rotated around the axial center C2 to lock the locking piece 94a to the locking part 92c. The opening edge 94b on the side opposite to the sensor attaching member 92 of the contact member 94 is to be brought into contact with a subject's face. Thus, the opening edge 94b conforms to the shape of the curve of a subject's face. The contact member 94 is formed of a material having an appropriate flexibility, such as rubber, resin or the like. Then, the contact member 94 is moderately pressed so as to bring the opening edge 94b itself into approximately close contact with the subject's face.

Specifically, the cover 90 brings the opening edge 94b into close contact with the skin around a subject's eye to form an enclosed space in front of the eye and places the detection surface 30 (sensor 40) so as to face to the eyeball in this enclosed space. The apparatus for measuring eye's moisture 2 according to this embodiment is configured to measure the evaporation amount of tear fluid from an eye (mainly from the surface of an eyeball) by using the sensor 40 to measure the amount of moisture (that is, humidity) in the enclosed space.

The sensor 40 in this embodiment has a similar structure to the sensor 40 shown in Fig. 4(b) except to have a larger size than the sensor 40 in the first embodiment (the length L of the one side of the substrate 42 is greater than that in the first embodiment). Therefore, this embodiment has a larger sensor 40 than the first embodiment so as to have a high sensitivity for detecting a change in humidity in the enclosed space. Moreover, In this embodiment, the detection sensitivity is further enhanced by increasing the number of the adjoining parts 44b and 46b (the number of comb teeth) than those shown in Fig. 4(b).

The sensor 40 may be disposed in a state in which the surface 42a having the first electrode 44 and the second electrode 46 formed thereon is covered with an appropriate resin or the like as Figs. 5(a) to 5(c) show in the first embodiment. However, the sensor 40 may be disposed in a state in which the first electrode 44 and the second electrode 46 are exposed because of a low possibility of short-circuit. Furthermore, a high-polymer humidity sensitive film which adsorbs moisture in air may be disposed on the surface 42a side of the sensor 40. Still furthermore, a protection wall may be disposed on the front side (subject's face side) of the sensor 40 to prevent an eyeball etc. from being in contact with the sensor 40.

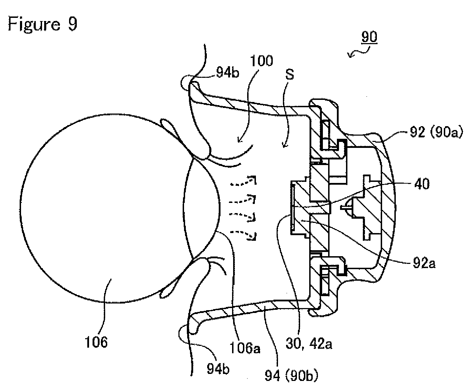

Next, the use of the apparatus for measuring eye's moisture 2 will be explained below. Fig. 9 is a diagram showing one example of a state during the use of the apparatus for measuring eye's moisture 2. Before the measurement of eye's moisture with the apparatus for measuring eye's moisture 2, a new contact member 94 (or a washed and sterilized contact member 94) is attached to the sensor attaching member 92. Subsequently, the operation button 80 of the main body 12 is operated to turn on the power supply of the apparatus for measuring eye's moisture 1 and switch to the measurement mode.

After the apparatus for measuring eye's moisture 2 is switched to the measurement mode, the measurer brings the opening edge 94b of the cover 90 into contact with a subject's face to cover the eye 100 with the cover 90 as Fig. 9 shows. As a result, the enclosed space S is formed in front of the eye 100. The detection surface 30 is placed at a position opposed mainly to the cornea 106a of the eyeball 106, and the surface 42a of the sensor 40 thus faces mainly to the cornea 106a.

Subsequently, the opening edge 94b of the cover 90 is maintained in contact with the subject's face for only a predetermined time (for example, from several seconds to several tens of seconds). In the meantime, the sensor 40 measures the amount of moisture in the enclosed space S, that is, the amount of tear fluid evaporated from the surface of the eyeball 106. Then, the measurement result and various indices or the like are displayed on the display 70.

It is a traditionally well-known fact that there is a correlation between the evaporation and the secretion of tear fluid. For example, the amount of tear fluid secretion can be judged to be small when the evaporation amount of tear fluid is small. Moreover, various states of tear fluid, such as the drainage state of tear fluid from the lachrymal punctum and the states of the mucus layer, the aqueous layer, and the oil layer in tear fluid on the surface of the eyeball 106 can be evaluated by measuring a temporal change in evaporation of tear fluid.

Particularly, since the detection surface 30 provided with the sensor 40 faces to the eyeball 106, this embodiment is capable of detecting the moisture evaporated from the surface of the eyeball 106 (that is, cornea 106a, sclera, and bulbar conjunctiva) with high sensitivity. This embodiment also can easily adjust the distance between the surface of the eyeball 106 and the detection surface 30 by adjusting of the projection amount of the projecting part 92a on which the detection surface 30 is provided. This allows the setting of an appropriate detection sensitivity, resulting in the shortening of the measurement time.

In this embodiment, only one cover 90 is provided to carry out the measurement of eyes one by one. However, two covers 90 are provided to carry out the measurement of eyes at one time. Moreover, the cover 90 may be fixed to a subject's face with such as a rubber band or the like. During measurement, the evaporation may be measured first with an eyelid closed, followed by another measurement of the evaporation with the eyelid opened. This can eliminate the influence of sweating from the eyelid or the like

As described above, the apparatus for measuring eye's moisture 1, 2 includes : a detection surface 30 placed on a position in contact with a conjunctiva, a sclera, or a cornea or a position opposed to a conjunctiva, a sclera, or a cornea; and a capacitance sensor 40 provided on the detection surface 30.

In the method for measuring eye's moisture, a detection surface 30 on which the capacitance sensor 40 is provided is located at a position in contact with a conjunctiva, a sclera, or a cornea or a position opposed to a conjunctiva, a sclera, or a cornea.

Such a structure can simply and objectively measure the state of tear fluid. Specifically, since the capacitance sensor 40 is adopted, the amount of eye's moisture can be measured with a high degree of accuracy, and the measurement result as well as indices or the like based on the measurement result can be displayed in objective numerical values only by placing the capacitance sensor 40 appropriately.

The apparatus for measuring eye's moisture 1 further includes a gripper 10 having an approximate-rod shape, the gripper 10 being connected with the detection surface 30, in which an angle θ between the longitudinal direction (axial center C1) of the gripper 10, and the detection surface 30 is from 0 to 45°. In this way, the detection surface 30 can be brought into contact with a conjunctiva or the like and measurements can be taken without a sense of fear on a subject.

The apparatus for measuring eye's moisture 1 further includes a detachable part 20 detachably connected with one end in the longitudinal direction of the gripper 10, in which the detection surface 30 is provided on the detachable part 20. In this way, the detection surface 30 that is brought into contact with a subject can be disposable, or cleaning and sterilization can be facilitated all around the detection surface 30, so as to keep proper hygienic condition.

In the apparatus for measuring eye's moisture 1, the detachable part 20 has a curved shape which gradually decreases its cross-sectional area from the gripper 10 to the detection surface 30. In this way, the detection surface 30 can be made smaller as much as possible, while the detection surface 30 can be brought into contact with the eye without a sense of fear on a subject.

In the apparatus for measuring eye's moisture 1, the detection surface 30 is disposed at an outer peripheral side of the outer peripheral surface of the gripper 10. In this way, the apparatus for measuring eye' s moisture 1 can be brought closer to a subject without a sense of fear on the subject, while the detection surface 30 can be brought into contact with a conjunctiva or the like smoothly and appropriately.

In the apparatus for measuring eye's moisture 1, the detection surface 30 has a circular or an elliptical shape. This can reduce the possibility of damage on a conjunctiva or the like and enhance the safety of the measurement.

The apparatus for measuring eye's moisture 1 further includes: a controller 50 controlling the sensor 40; a display 70 displaying a measurement result from the sensor 40; and a power supply 60 supplying electric power to the controller 50 and the display 70, in which the controller 50, the display 70, and the power supply 60 are provided in the gripper 10. This makes it simple to handle the apparatus for measuring eye's moisture 1 and allows the measurement in various places, such as home and outdoor.

The apparatus for measuring eye's moisture 2 further includes: a cover 90 having an approximate-cup shape, the cover 90 being disposed so as to bring the opening edge 94b of the cover 90 into contact with a subject's face, in which the detection surface 30 is provided inside the cover 90. In this way, the state of tear fluid can be determined without bringing the detection surface 30 into contact with a conjunctiva or the like, allowing measurements to be taken regardless of the condition of eyes. Furthermore, the apparatus for measuring eye's moisture 2 provides different types of information from what the apparatus for measuring eye's moisture 1 provide, which allows more broad-ranging evaluation of tear fluid.

In the apparatus for measuring eye's moisture 2, the detection surface 30 is provided at the bottom 90a of the cover 90. In this way, the evaporation amount of tear fluid can efficiently be measured with the sensor 40.

In the apparatus for measuring eye's moisture 2, the cover 90 is provided with a sensor attaching member 92 to which the sensor 40 is attached; and a contact member 94 to be brought into contact with a subject's face, in which the sensor attaching member 92 and the contact member 94 are detachably connected with each other. In this way, the contact member 94 that is brought into contact with a subject can be disposable, or cleaning and sterilization of the contact member 94 can be facilitated, so as to keep proper hygienic conditions.

The sensor 40 is provided with a substrate 42 disposed along the detection surface 30; and a first electrode 44 and a second electrode 46, both of which are formed on the same face of the substrate 42, in which at least one linear gap 48 is provided between the first electrode 44 and the second electrode 46. In this way, the sensor 40 can be downsized while appropriate detection sensitivity can be secured. As a result, the detection surface 30 can be downsized, and thus the number of sites accessible to the detection surface 30 can be increased to carry out unprecedentedly precise measurement and evaluation.

The first electrode 44 and the second electrode 46 are provided with adjoining parts 44b, 46b adjoining across the linear gap 48, respectively, and the adjoining parts 44b, 46b are each formed in a shape having a width W in a distance CL direction of the linear gap 48 that is greater than the distance CL of the linear gap 48. Since this allows both downsizing and improvement in the detection sensitivity of the sensor 40, broad-ranging measurement of eyes can be carried out with a high degree of accuracy.

The embodiments of the present invention are described above. However, the apparatus for measuring eye's moisture or the method for measuring eye's moisture of the present invention is not limited to the above-mentioned embodiments. Needless to say, various modifications may be made without departing from the spirit and scope of the present invention.

For example, the shape of the members such as the gripper 10, the main body 12, the detachable part 20, and the cover 90 is not limited to those described in the above-mentioned embodiments. The members may have any shape. Depending on the shape of the gripper 10 and the detachable part 20, the apparatus for measuring eye's moisture 1 may be moved in a direction approximately parallel to the axial center C1 of the gripper 10 so as to bring the detection surface 30 into contact with a conjunctiva or the like. The shape of the substrate 42 of the sensor 40 may be such as rectangle or the like. The first electrode 44 and the second electrode 46 may have appropriate shape other than those described in the above-mentioned embodiments.

The attachment of the detachable part 20 to gripper 10 and the connection of the sensor attaching member 92 with the contact member 94 may be made by a method such as screwing, clamping, or press fitting other than locking. The sensor attaching member 92 is not limited to form only the bottom 90a of the cover 90. The sensor attaching member 92 may form a part of the bottom 90a and the skirt 90b. In this case, the sensor 40 may be disposed on the skirt 90b.

The gripper 10 may be connected with the cover 90. Specifically, a same gripper 10 may be used for the apparatus for measuring eye's moisture 1, 2 by appropriately having a controller 50 and the like. In this case, the cover 90 may be attached to the gripper 10 without the cable 14.

The gripper 10 and the main body 12 may be provided with a communication part sending and receiving data to an outside computer or the like through wireless or wired communication. In the apparatus for measuring eye's moisture 1, the controller 50, the power supply 60, the display 70, and the operation button 80 may be provided outside the gripper 10 and may be composed of an existing computer. In the apparatus for measuring eye's moisture 2, the main body 12 also may be composed of an existing computer.

Depending on the performance, the structure or the like of the sensor 40, a plurality of sensors 40 may be arranged on the detection surface 30, and a plurality of detection surfaces 30 may be provided. For example, in the apparatus for measuring eye's moisture 1, two detection surfaces 30 may be brought into contact with both eyes at the same time. In the apparatus for measuring eye's moisture 2, a plurality of sensors 40 maybe placed opposed to the corresponding plurality of sites on the surface of an eyeball.

The workings and the effects that the above-mentioned embodiments show are merely recited as most suitable examples of the present invention. Therefore, the working and the effect of the present invention are not limited to these.

### Industrial Applicability

The apparatus and the method for measuring eye's moisture of the present invention can be applied to various fields relating to eyes, such as the development of various eye-drops, eyewear or the like and daily eye health care in addition to field of ophthalmology such as the diagnosis of dry eye and the selection of contact lens.

### Reference Signs List

- 1, 2: apparatus for measuring eye's moisture
- 10: gripper
- 20: detachable part
- 30: detection surface
- 40: sensor
- 42: substrate
- 44: first electrode
- 46: second electrode
- 48: linear gap
- 50: controller
- 60: power supply
- 70: display
- 90: cover
- 90a: bottom of cover
- 92: sensor attaching member
- 94: contact member
- 94b: opening edge of cover
- C1: longitudinal axial center of gripper
- CL: interval between adjoining parts (distance of gap)
- W: electrode width (dimension in distance direction of gap)
- θ: angle between longitudinal axial center of gripper and detection surface

## Claims

1. An apparatus for measuring eye's moisture, comprising: a detection surface placed on a position in contact with a conjunctiva, a sclera, or a cornea or a position opposed to a conjunctiva, a sclera, or a cornea; and a capacitance sensor provided on the detection surface.

2. The apparatus for measuring eye's moisture according to claim 1, further comprising: a gripper having an approximate-rod shape, the gripper being connected with the detection surface, wherein an angle between the longitudinal direction of the gripper and the detection surface is from 0 to 45°.

3. The apparatus for measuring eye's moisture according to claim 2, further comprising a detachable part detachably connected with one end in the longitudinal direction of the gripper, wherein the detection surface is provided on the detachable part.

4. The apparatus for measuring eye's moisture according to claim 3, wherein the detachable part has a curved shape which gradually decreases its cross-sectional area from the gripper to the detection surface.

5. The apparatus for measuring eye's moisture according to any one of claims 2 to 4, wherein the detection surface is disposed at an outer peripheral side of the outer peripheral surface of the gripper.

6. The apparatus for measuring eye's moisture according to any one of claims 2 to 5, wherein the detection surface has a circular or an elliptical shape.

7. The apparatus for measuring eye's moisture according to any one of claims 2 to 6, further comprising: a controller controlling the sensor; a display displaying a measurement result by the sensor; and a power supply supplying electric power to the controller and the display, wherein the controller, the display, and the power supply are provided in the gripper.

8. The apparatus for measuring eye's moisture according to claim 1, further comprising: a cover having an approximate-cup shape, the cover being disposed so as to bring the opening edge of the cover into contact with a subject's face, wherein the detection surface is provided inside the cover.

9. The apparatus for measuring eye's moisture according to claim 8, wherein the detection surface is provided at the bottom of the cover.

10. The apparatus for measuring eye's moisture according to claim 8 or 9, wherein the cover is provided with a sensor attaching member to which the sensor is attached; and a contact member to be brought into contact with a subject's face, wherein the sensor attaching member and the contact member are detachably connected with each other.

11. The apparatus for measuring eye's moisture according to any one of claims 1 to 10, wherein the sensor is provided with a substrate disposed along the detection surface; and a first electrode and a second electrode, both of which are formed on the same face of the substrate, wherein at least one linear gap is provided between the first electrode and the second electrode.

12. The apparatus for measuring eye's moisture according to claim 11, wherein the first electrode and the second electrode are each provided with an adjoining part, the adjoining parts adjoining across the linear gap, and the adjoining part is formed in a shape having a width in a distance direction of the linear gap that is greater than the distance of the linear gap.

13. A method for measuring eye's moisture, wherein a detection surface on which a capacitance sensor is provided is located at a position in contact with a conjunctiva, a sclera, or a cornea or a position opposed to a conjunctiva, a sclera, or a cornea.
